# EUROPÄISCHE PATENTANMELDUNG

(11) **EP 1 153 596 A2**
(43) Veröffentlichungstag der Anmeldung: **14.11.2001**
(21) Anmeldenummer: 01110631.7
(22) Anmeldetag: 30.04.2001
(51) Int. Cl.: A61K 7/08, A61K 7/11

(54) **Haarbehandlungsmittel**

(30) Priorität: 12.05.2000 DE 10023334
(71) Anmelder: Clariant GmbH, 65929 Frankfurt am Main (DE)
(72) Erfinder: Klein, Sonja, 65795 Hattersheim (DE)
(74) Vertreter: Mikulecky, Klaus

(57) **Zusammenfassung**

Die Erfindung betrifft wässrige Haarbehandlungsmittel, enthaltend eine oder mehrere in Wasser schwerlösliche quaternäre Ammoniumverbindungen, die mindestens eine langkettige Alkyl- oder Alkenylgruppe mit mindestens 18 Kohlenstoffatomen enthalten, eine oder mehrere wasserlösliche quaternäre Ammoniumverbindungen, einen oder mehrere nichtionische, amphotere und/oder zwitterionische Lösungsvermittler und gegebenenfalls ein oder mehrere Verdickungsmittel. Die erfindungsgemäßen Mittel zeichnen sich durch ein klares und durchscheinendes Aussehen aus. Insbesondere handelt es sich bei den Mitteln um Haarkuren und Haarspülungen.

## Beschreibung

Die Erfindung betrifft Haarbehandlungsmittel zum Pflegen und Glätten der Haare. Die erfindungsgemäßen Mittel zeichnen sich durch ein klares und durchscheinendes Aussehen aus.

Haarbehandlungsmittel zum Konditionieren von menschlichen Haaren sind seit langem bekannt. Sie enthalten in der Regel in Wasser schwerlösliche quaternäre Ammoniumverbindungen, die mindestens eine langkettige Alkyl- oder Alkenylgruppe aufweisen. Solche Mittel sind üblicherweise als wässrige Dispersionen bzw. Emulsionen, Mikroemulsionen, Gele oder auch in Aerosolform konfektioniert und werden als Haarkuren, Harrspülungen etc. eingesetzt.
Nachteilig an derartigen Mitteln ist jedoch, dass sie aufgrund der schwerlöslichen quaternären Ammoniumverbindungen kein klares Aussehen zeigen, sondern durch ihre Trübung stumpf wirken und ästhetisch wenig ansprechend sind.

Zielsetzung war es somit, wässrige Haarbehandlungsmittel zu entwickeln, die ein klares und durchscheinendes Aussehen haben.

Überraschenderweise wurde nun gefunden, dass wässrige Haarbehandlungsmittel, enthaltend als Komponenten
A) eine oder mehrere in Wasser schwerlösliche quaternäre Ammoniumverbindungen, die mindestens eine langkettige Alkyl- oder Alkenylgruppe mit mindestens 18 Kohlenstoffatomen enthalten,
B) eine oder mehrere wasserlösliche quaternäre Ammoniumverbindungen,
C) einen oder mehrere nichtionische, amphotere und/oder zwitterionische Lösungsvermittler und
D) gegebenenfalls ein oder mehrere Verdickungsmittel, ein klares und durchscheinendes Aussehen haben.

Bevorzugt enthalten die erfindungsgemäßen Haarbehandlungsmittel, bezogen auf das fertige Haarbehandlungsmittel,
0,1 bis 10 Gew.-%, besonders bevorzugt 0,2 bis 5 Gew.-%, insbesondere bevorzugt 0,5 bis 1,5 Gew.-% der Komponente A),
0,1 bis 20 Gew.-%, besonders bevorzugt 0,5 bis 10 Gew.-%, insbesondere bevorzugt 1 bis 5 Gew.-% der Komponente B),
0,1 bis 20 Gew.-%, besonders bevorzugt 0,5 bis 10 Gew.-%, insbesondere bevorzugt 1 bis 5 Gew.-% der Komponente C) und
0,1 bis 15 Gew.-%, besonders bevorzugt 0,3 bis 6 Gew.- %, insbesondere bevorzugt 0,5 bis 3 Gew.- % der Komponente D).

Als in Wasser schwerlösliche quaternäre Ammoniumverbindungen A) eignen sich bevorzugt Behenyltrimethylammoniumchlorid, Stearyltrimethylammoniumchlorid und/oder Distearyldimethylammoniumchlorid.
Besonders bevorzugt ist Behenyltrimethylammoniumchlorid.

Als wasserlösliche quarternäre Ammoniumverbindungen B) eignen sich bevorzugt Lauryltrimethylammoniumchlorid, Cetyltrimethylammoniumchlorid und/oder PEG 5 Stearylammoniumlactat. Besonders bevorzugt eignen sich Cetyltrimethylammoniumchlorid und/oder PEG 5 Stearylammoniumlactat.

Als Lösungsvermittlern C) geeignet sind nichtionische, amphotere und/oder zwitterionische Tenside.

Als nichtionische Tenside bevorzugt geeignet sind Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics® ); Fettsäurealkylolamide (Fettsäureamidpolyethylenglykole); N-Alkyl- und N-Alkoxy-polyhydroxyfettsäureamide; Zuckerester (z.B. Saccharoseester, Sorbitester) und Zuckerether (z.B. Glucoseether); und/oder Polyglykolether.

Als amphotere Tenside bevorzugt geeignet sind Aminoxide, insbesondere bevorzugt sind (C₁₂-C₁₈)-Alkyldimethylaminoxide und Fettsäureamidoalkyldimethylaminoxide, ganz besonders bevorzugt sind das Lauryldimethylaminoxid und das Cocaminoxid; N-(C₁₂-C₁₈-Alkyl)-β-aminopropionate und N-(C₁₂-C₁₈-Alkyl)-β-iminodipropionate in Form ihrer Alkali- und Mono-, Di- und Trialkylammoniumssalze; N-Acylamidoalkyl-N,N-dimethylacetobetaine, bevorzugt die N-(C₈-C₁₈-Acyl)amido-propyl-N,N-dimethylacetobetaine; (C₁₂-C₁₈)-Alkyl-dimethylsulfopropylbetaine; und/oder Amphotenside auf Basis von Imidazolinen (z.B. Miranol® und Steinapon® ), bevorzugt das Natriumsalz des 1-(β-Carboxy-methyloxyethyl)-1-(carboxymethyl)-2-laurylimidazolins.

Als zwitterionische Tenside bevorzugt geeignet sind Alkylamidopropylbetaine, besonders bevorzugt das Cocamidopropylbetain; N-Alkyl-N,N-dimethylammoniumglycinate, z.B. Kokosalkyldimethylammoniumglycinate; N-Acyl-aminopropyl-N-N-dimethylammoniumglycinate, z.B. Kokosacylaminopropyldimethylammoniumglycinate; 2-Alkyl-3-carboxymethyl-3-hydroxyethylimidazoline mit jeweils 8 bis 18 C-Atomen in der Alkyl- oder Acylgruppe; und/oder das Kokosacylaminoethylhydroxyethylcarboxymethylglycinat.

Als Lösungsvermittler C) besonders bevorzugt sind Aminoxide, insbesondere bevorzugt das Lauryldimethylaminoxid und/oder das Cocaminoxid.

Als Verdickungsmittel D) bevorzugt geeignet sind Hydroxyethylcellulose; Carboxymethylcellulose; Polysaccharide, wie z.B. Xantham-Gum, Guar-Guar, Agar-Agar, Alginate, PEG-120 Methyl Glucose Dioleate, Methylglucose; Dioleate; höhermolekulare Polyethylenglykolmono- und -diester von Fettsäuren; Polyacrylate; Polyvinylalkohole; Polyvinylpyrrolidone; Tenside, wie z.B. ethoxylierte Fettsäureglyceride, Ester von Fettsäuren mit Polyolen (z.B. Pentaerythrit), Fettalkohole, Fettalkoholethoxylate, Alkyloligoglucoside; und/oder Isopropanolamidmischungen auf Cocosfettsäurebasis.
Als Verdickungsmittel insbesondere geeignet ist Hydroxyethylcellulose.

Die erfindungsgemäßen Haarbehandlungsmittel können weitere Zusatz- und Hilfsstoffe, wie Ölkörper, Überfettungsmittel, Stabilisatoren, Konsistenzgeber, Kationpolymere, Siliconverbindungen, biogene Wirkstoffe, Antischuppenmittel, Filmbildner, Konservierungsmittel, Säuren, Laugen, Puffer, Hydrotrope, feuchtigkeitsspendende Substanzen, Solubilisatoren, UV-Absorber, Farbstoffe und/oder Duftstoffe enthalten.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12-24 C-Atomen, Fettsäureester mit insgesamt 12-26 C-Atomen, flüssige Kohlenwasserstoffe mit 10-32 C-Atomen und Gemische davon. Geeignete Fettsäureester sind z.B. Methylpalmitat, Ethyloleat, Isopropylmyristat, n-Hexyllaurat, n-Butylstearat, Cetyl- und Stearylisononanoat. Besonders bevorzugt sind Paraffinöle, Vaseline, Pflanzenöle, synthetische Triglyceride, wie z.B. Glyceryltricaprylat, sowie Silikonöle.

Als Überfettungsmittel können Substanzen wie beispielsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Geeignete Siliconverbindungen sind beispielsweise Dimethylpolysiloxan, Methylphenylpolysiloxane, cyclische Silicone und/oder amino-, fettsäure-, alkohol-, polyether-, epoxy-, fluor- und/oder alkylmodifizierte Siliconverbindungen, die bei Raumtemperatur sowohl flüssig als auch harzförmig vorliegen können.

Als biogene Wirkstoffen eignen sind beispielsweise Bisabolol® , Allantoin® , Phytantriol® , Panthenol® , AHA-Säuren, Pflanzenextrakte und Vitaminkomplexe.

Als Antischuppenmittel können Climbazol® , Octopirox® , Oxiconazol® und Zinkpyrethion® eingesetzt werden.

Gebräuchliche Filmbildner sind Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als Säuren bzw. Laugen zur pH-Wert Einstellung werden bevorzugt Zitronensäure und/oder Natronlauge verwendet.

Zur Verbesserung des Fließverhaltens können Hydrotrope, wie beispielsweise Ethanol, Isopropylalkohol, Propylenglykol oder Glucose, eingesetzt werden.

Als feuchtigkeitsspendende Substanz stehen beispielsweise Isopropylpalmitat, Glycerin und/ oder Sorbitol zu Verfügung.

Als Konservierungsmittel eignen sich beispielsweise Phenoxyethanol, Formaldehydlösung, Parabene, Pentandiol oder Sorbinsäure.

Der Gesamtanteil an Hilfs- und Zusatzstoffen in den Haarbehandlungsmitteln beträgt bevorzugt 1 bis 30 Gew.-%, besonders bevorzugt 2 bis 20 Gew.-%.

Bei den erfindungsgemäßen Haarbehandlungsmitteln handelt es sich bevorzugt um Haarkuren und Haarspülungen.

Bevorzugt werden die erfindungsgemäßen Haarbehandlungsmittel so hergestellt, dass die Komponenten A), B) und C) bei Temperaturen von 45-85°C, bevorzugt 55-75°C, unter Rühren in Wasser gelöst werden und die Mischung anschließend auf Raumtemperatur abgekühlt wird und gegebenenfalls anschließend der Mischung ein oder mehrere Verdickungsmittel D) in Form einer wässrigen Dispersion bzw. Lösung zugegeben werden.

### Beispiele

In den folgenden Beispielen werden Formulierungen für erfindungsgemäße Haarsprühkuren (Beispiele 1 und 2) und Haarspülungen (Beispiele 3 bis 10) angegeben. Die Beispiele dienen der näheren Erläuterung der Erfindung ohne sie jedoch einzuschränken. Alle Prozentangaben sind Gewichtsprozente.

Neben den in den jeweiligen Beispielen angegebenen Komponenten enthalten die Formulierungen noch Zitronensäure und geringe Mengen Parfumöl, Konservierungsmittel und Farbstofflösung ad 100 Gew.-% der Formulierung.

### Haarsprühkuren 1 und 2:

Zur Herstellung der Haarsprühkuren 1 und 2 wird jeweils eine Mischung aus den Komponenten I) und II) hergestellt. Dazu wird die Komponente I) bei ca. 60°C unter Rühren in der Komponente II) bis zum Klarwerden gelöst. Anschließend wird die Mischung auf Raumtemperatur abgekühlt. Danach wird der pH-Wert mittels Zitronensäure auf ca. pH=4 eingestellt.

| | Haarsprühkur 1: | |
|---|---|---|
| I) | Genamin CTAC | 5 % |
| | Genamin KDMP | 1 % |
| | Genaminox CS | 4 % |
| | Cetiol HE | 2 % |
| | Panthenol | 0.2 % |
| II) | Wasser | ad 100 % |

| | Haarsprühkur 2: | |
|---|---|---|
| I) | Genamin KDMP | 1% |
| | Genamin KSL | 5 % |
| | Genaminox CS | 4 % |
| | Cetiol HE | 2 % |
| | Panthenol | 0.2 % |
| II) | Wasser | ad 100 % |

Haarspülungen 3 bis 10:

Zur Herstellung der Haarspülungen 3 bis 10 wird jeweils eine Mischung 1 aus den Komponenten I) und II) hergestellt. Dazu wird die Komponente I) bei ca. 60°C unter Rühren in der Komponente II) bis zum Klarwerden gelöst. Anschließend wird die Mischung 1 auf Raumtemperatur abgekühlt. Zur Herstellung einer Mischung 2 wird jeweils die Komponente III) in der Komponente IV) dispergiert und die Mischung bis zum Klarwerden gerührt. Anschließend werden die Mischungen 1 und 2 unter Rühren miteinander vermischt. Danach wird der pH-Wert mittels Zitronensäure auf ca. pH=4 eingestellt.

| | Haarspülung 3: | |
|---|---|---|
| I) | Genamin CTAC | 5,0 % |
| | Genamin KDMP | 1,0 % |
| | Genapol DU-080 | 1,0 % |
| | Genagen CA-050 | 1,0 % |
| II) | Wasser | 40,0 % |
| III) | Tylose H 10000 G4 | 2,0 % |
| IV) | Wasser | ad 100 % |

| | Haarspülung 4: | |
|---|---|---|
| I) | Genamin CTAC | 5,0 % |
| | Genamin KDMP | 0,5 % |
| | Genaminox LA | 4,0 % |
| | Cetiol HE | 2,0 % |
| II) | Wasser | 37,7 % |
| III) | Tylose H 100000YP2 | 0,8 % |
| IV) | Wasser | ad 100 % |

| | Haarspülung 5: | |
|---|---|---|
| I) | Genamin CTAC | 5,0 % |
| | Genamin KDMP | 0,5 % |
| | Genaminox LA | 5,0 % |
| | Velsan D8P-3 | 1,0 % |
| II) | Wasser | 27,3 % |
| III) | Tylose H | 1,2% |
| IV) | Wasser | ad 100 % |

| | Haarspülung 6: | |
|---|---|---|
| I) | Genamin KSL | 5,0 % |
| | Genamin KDMP | 1,0 % |
| | Genaminox KC | 6,0 % |
| II) | Wasser | 36,8 % |
| III) | Tylose H | 1,2% |
| IV) | Wasser | ad 100 % |

| | Haarspülung 7: | |
|---|---|---|
| I) | Genamin CTAC | 5,0 % |
| | Genamin KDMP | 1,0% |
| | Genaminox CS | 6,0 % |
| | Cetiol HE | 2,0 % |
| II) | Wasser | 36,8 % |
| III) | Tylose H | 1,2% |
| IV) | Wasser | ad 100 % |

| | Haarspülung 8: | |
|---|---|---|
| I) | Genamin CTAC | 5,0 % |
| | Genamin KSL | 5,0 % |
| | Genamin KDMP | 1,0 % |
| | Genaminox CS | 6,0 % |
| | Cetiol HE | 2,0 % |
| | Vitamin E | 0,2 % |
| | Vitamin B 5 | 0,7 % |
| II) | Wasser | 36,8 % |
| III) | Tylose H | 1,5% |
| IV) | Wasser | ad 100 % |

| | Haarspülung 9: I) Genamin CTAC | 5,0 % |
|---|---|---|
| | Genamin STAC | 0,5 % |
| | Genaminox LA | 4,0 % |
| | Cetiol HE | 2,0 % |
| II) | Wasser | 37,7 % |
| III) | Tylose H 100000YP2 | 0,8 % |
| IV) | Wasser | ad 100 % |

| | Haarspülung 10: | |
|---|---|---|
| I) | Genamin CTAC | 5,0 % |
| | Genamin DSAC | 0,5 % |
| | Genaminox LA | 4,0 % |
| | Cetiol HE | 2,0 % |
| II) | Wasser | 37,7 % |
| III) | Tylose H 100000YP2 | 0,8 % |
| IV) | Wasser | ad 100 % |

| INCI Bezeichnung der eingesetzten Handelsprodukte: | | |
|---|---|---|
| Genamin KDM-P® | (Clariant GmbH) | Behenyltrimethylammonium Chlorid |
| Genamin KSL® | (Clariant GmbH) | PEG- 5 Stearyl Ammonium Lactat |
| Genamin CTAC® | (Clariant GmbH) | Cetyltrimethylammonium Chlorid |
| Genamin STAC® | (Clariant GmbH) | Stearyltrimethylammonium Chlorid |
| Genamin DSAC® | (Clariant GmbH) | Distearyldimethylammonium Chlorid |
| Tylose H 100000 YP2® | (Clariant GmbH) | Hydroxyethylcellulose |
| Tylose H | (Clariant GmbH) | Hydroxyethylcellulose |
| Genaminox La | (Clariant GmbH) | Lauryldimethylaminoxid |
| Genaminox KC | (Clariant GmbH) | Cocaminoxid |
| Genaminox CS | (Clariant GmbH) | Cocaminoxid |
| Genapol DU-80 | (Clariant GmbH) | Undeceth-8 |
| Velsan D8P-3 (Clariant GmbH) | | Isopropyl PPG-2-lsodeceth-7 Carboxylat |
| Cetiol HE (Henkel) | | PEG-7 Glyceryl Cocoat |
| Genagen CA-050 (Clariant GmbH) | | PEG-5 Cocamid |

## Patentansprüche

1. Wässriges Haarbehandlungsmittel, enthaltend als Komponenten
A) eine oder mehrere in Wasser schwerlösliche quaternäre Ammoniumverbindungen, die mindestens eine langkettige Alkyl- oder Alkenylgruppe mit mindestens 18 Kohlenstoffatomen enthalten,
B) eine oder mehrere wasserlösliche quaternäre Ammoniumverbindungen,
C) einen oder mehrere nichtionische, amphotere und/oder zwitterionische Lösungsvermittler und
D) gegebenenfalls ein oder mehrere Verdickungsmittel.

2. Haarbehandlungsmittel nach Anspruch 1, **dadurch gekennzeichnet, dass** es, bezogen auf das fertige Mittel, 0,1 bis 10 Gew.-% der Komponente A), 0,1 bis 20 Gew.-% der Komponente B), 1 bis 5 Gew.-% der Komponente C) und 0,1 bis 15 Gew.-% der Komponente D) enthält.

3. Haarbehandlungsmittel nach Anspruch 1 und/oder Anspruch 2, **dadurch gekennzeichnet, dass** es als Komponente A) Behenyltrimethylammoniumchlorid, Stearyltrimethylammoniumchlorid und/oder Distearyldimethylammoniumchlorid enthält.

4. Haarbehandlungsmittel nach mindestens einem der Ansprüche 1 bis 3, **dadurch gekennzeichnet, dass** es als Komponente A) Behenyltrimethylammoniumchlorid enthält.

5. Haarbehandlungsmittel nach mindestens einem der Ansprüche 1 bis 4, **dadurch gekennzeichnet, dass** es als Komponente B) Lauryltrimethylammoniumchlorid, Cetyltrimethylammoniumchlorid und/oder PEG 5 Stearylammoniumlactat enthält.

6. Haarbehandlungsmittel nach mindestens einem der Ansprüche 1 bis 5, **dadurch gekennzeichnet, dass** es als Lösungsvermittler C) Aminoxide enthält.

7. Haarbehandlungsmittel nach Anspruch 6 **dadurch gekennzeichnet, dass** es sich bei den Aminoxiden um Lauryldimethylaminoxid und/oder Cocaminoxid handelt.

8. Haarbehandlungsmittel nach mindestens einem der Ansprüche 1 bis 7, **dadurch gekennzeichnet, dass** es als Verdickungsmittel D) Hydroxyethylcellulose enthält.

9. Haarbehandlungsmittel nach mindestens einem der Ansprüche 1 bis 8, **dadurch gekennzeichnet, dass** es gegebenenfalls noch Ölkörper, Überfettungsmittel, Stabilisatoren, Konsistenzgeber, Kationpolymere, Siliconverbindungen, biogene Wirkstoffe, Antischuppenmittel, Filmbildner, Konservierungsmittel, Säuren, Puffer, Hydrotrope, feuchtigkeitsspendende Substanzen, Solubilisatoren, UV-Absorber, Farbstoffe und/oder Duftstoffe enthält.

10. Haarbehandlungsmittel nach mindestens einem der Ansprüche 1 bis 9, **dadurch gekennzeichnet, dass** es sich dabei um eine Haarkur oder eine Haarspülung handelt.

11. Verfahren zur Herstellung eines Haarbehandlungsmittels entsprechend mindestens einem der Ansprüche 1 bis 9, **dadurch gekennzeichnet, dass** die Komponenten A), B) und C) bei Temperaturen von 45 - 85°C unter Rühren in Wasser gelöst werden und die Mischung anschließend auf Raumtemperatur abgekühlt wird und gegebenenfalls anschließend der Mischung ein oder mehrere Verdickungsmittel D) in Form einer wässrigen Dispersion bzw. Lösung zugegeben werden.
